# EUROPEAN PATENT APPLICATION

(11) **EP 4 755 306 A1**
(43) Date of publication of application: **10.06.2026**
(21) Application number: 24217365.6
(22) Date of filing: 04.12.2024
(51) Int. Cl.: A61B 6/03, A61B 6/40, A61B 6/00

(54) **SLOW KVP MODULATION FOR CT**

(71) Applicant: Koninklijke Philips N.V., 5656 AE Eindhoven (NL)
(72) Inventor: DAERR, Heiner, Eindhoven (NL); MEINEKE, Jan Jakob, Eindhoven (NL)
(74) Representative: Philips Intellectual Property & Standards

(57) **Abstract**

Proposed concepts thus aim to provide schemes, solutions, concept, designs, methods and systems pertaining to operating a CT system. In particular, embodiments aim to a method for operating a CT system. This can be achieved by slowly varying a kVp level of an X-ray tube of the CT system (as opposed to having to use an expensive fast-switching kVp generator, for example) so that each voxel of the scanned volume is scanned/imaged under at least n different kVp levels, where n is 2 or more. In other words, by slowly varying the kVp level of an X-ray tube during rotation of the gantry of the CT system, every voxel of a scanned volume will be seen under at least n different kVp levels, thus enabling spectral CT imaging without any specialized hardware, thus facilitating spectral imaging even in a standard, one source, one detector CT system.

## Description

### FIELD OF THE INVENTION

This invention relates to the field of operating a CT system.

### BACKGROUND OF THE INVENTION

Spectral CT is becoming ever more adopted in clinical practice, and, for some applications, might even become the standard of care. Further, clinical guidelines may consider spectral CT for diagnosing specific clinical questions like calcium scoring, kidney stones characterization or cancer staging.

Spectral imaging will soon be available in the performance CT sector, no longer being limited to the premium CT sector. However, spectral imaging is still not enabled in the lower performance CT sector and though enabled in the upper value CT sector, it is high-cost. Current spectral CT systems are equipped with expensive hardware such as photon-counting detectors, fast kVp generators and tubes and/or dual layer detectors, providing a significant barrier to the wide-availability of spectral CT.

### SUMMARY OF THE INVENTION

The invention is defined by the claims.

According to examples in accordance with an aspect of the invention, there is provided a method for operating a CT system.

The invention enables the acquisition of spectral projection data even with a standard `single-energy' CT system without any (or with very minor) hardware changes. The core of the invention is to slowly vary the kVp level of a single X-ray tube in such a way that each image voxel is seen under at least 2 different kVp levels (and typically many more levels as voxels in the center of the volume/image will be seen by many different rays).

The method comprises: during a single rotation of a gantry of the CT system, slowly varying a kVp level of an X-ray tube of the CT system but still fast enough such that each voxel of a scanned volume is scanned under at least two different kVp levels. In one embodiment, a monotonic rate of change of the kVp level when falling is at most 5kVp/ms. In another embodiment, a monotonic rate of change of the kVp level when rising is at most 5kVp/ms.

Proposed concepts thus aim to provide schemes, solutions, concepts, designs, methods and systems pertaining to operating a CT system. In particular, embodiments aim to provide a method for operating a CT system by slowly varying a kVp level of an X-ray tube of the CT system (as opposed to having to use an expensive fast-switching kVp generator, for example) so that each voxel of the scanned volume is scanned/imaged under at least two different kVp levels. It should be noted that voxels in the center of the volume will be scanned under many different kVp levels as they are seen by many different rays, however, what is important is that all voxels are scanned under (at least) two kVp levels as this then enables material decomposition into (at least) two different materials. This slow variation is to be understood as a monotonic/uni-directional rate of change (for example, between two extrema kVp levels, i.e., maximum to minimum, or minimum to maximum) of at most 5kVp/ms. It therefore does not include the essentially instantaneous switching of a fast-switching kVp generator or switching between two different X-ray tubes of different energies. Instead, a single standard X-ray tube is used to smoothly vary the kVp value, thus facilitating spectral CT imaging with entirely standard (and thus cheaper and more available) equipment.

In other words, by slowly varying the kVp level of an X-ray tube during a whole rotation or at least during a substantial part of a rotation of the gantry of the CT system, every voxel of a scanned volume can be seen under at least two different kVp levels, thus enabling spectral CT imaging without any specialized hardware, thus facilitating spectral imaging even in a standard, one source, one detector CT system.

This invention thus provides a low-cost and more easily available solution for spectral CT imaging, and as such, would be of significant use in many different clinical applications.

The inventors have realized that the present invention can address the present and future need of a low-cost spectral CT solution, which provides a better time resolution than the known Spin-Spin solution (referring to the acquisition of two separate CT scans with a time delay of several seconds, strongly limiting the application space to non-contrasted scans). For example, the present invention can enable material decomposition with a temporal resolution of about half the gantry rotation time, e.g., around 125ms to 165ms for gantry rotation times of 0.25 and 0.33 seconds respectively.

Ultimately, an improved method for operating a CT system is provided by slowly varying a kVp level of the CT system's X-ray tube but still fast enough that every voxel in a volume to be scanned will be seen under at least two different kVp levels thus facilitating spectral imaging and so enabling material decomposition.

In a typical embodiment, the slow variation is at most 5kVp/ms.

In some embodiments, the CT system may be a single-source and single-detector CT system. This enables the method to be used even on the cheapest and most widely-available CT systems, where the present invention will have the most benefit.

In some embodiments, the variation of the kVp level may comprise a period of decreasing and a period of increasing, and the period of decreasing may be longer than the period of increasing. Decreasing the kVp level of an X-ray tube is dependent on the emission current, and thus its rate of change is limited compared to increasing the kVp level, which is essentially independent of the emission current. By letting the kVp level slowly decrease but then more rapidly increasing the kVp level back up to the maximum, for example, the number of views obtained with a similar kVp may be reduced, thus increasing the efficiency and utility of the scan.

In some embodiments, the kVp level of the X-ray tube may be varied in a sawtooth pattern. This may be a simple pattern to implement for the varying of the kVp level while providing a more uniform variation of different kVp levels.

In some embodiments, the kVp level may vary by at least 10kVp. This may ensure a minimum variation of kVp levels, thus facilitating more useful spectral imaging and hence more useful material decomposition.

In some embodiments, the kVp level of the X-ray tube may be varied in a pattern with a frequency that is different to the frequency of the gantry rotation. This may enable better sampling with different kVp levels.

In some embodiments, the method may further comprise using an adjustable filter in a collimator box of the CT system to controllably filter beams of different kVp levels. This minor hardware modification may further improve the spectral performance of the proposed method and reduce patient X-ray dose.

In some embodiments, the single rotation of the gantry of the CT system may be during a helical scan or an axial scan. These are both types of CT scans where the present invention would be of benefit.

In some embodiments, the method may further comprise generating projection data based on the scanned volume. This projection data may then be reconstructed, for example.

In some embodiments, the method may further comprise processing a first portion of the projection data, corresponding to pairs of detector measurements along the same path but in opposite directions, with a projection-based material decomposition algorithm to decompose the first portion of the projection data into at least two different materials. In other words, for a pair of detector measurements transversing the same path in opposite directions, also known as pi-lines or pi-partners, projection-based decomposition may be applied directly, facilitating more efficient material decomposition.

In some embodiments, the method may further comprise processing a second portion of the projection data with an iterative spectral reconstruction algorithm to decompose the second portion of the projection data into at least two different materials. This may enable all detector measurements to be used for material decomposition, regardless of whether they are pi-lines or pi-partners.

In some embodiments, processing the second portion of the projection data may be at least partly based on the decomposition of the first portion of the projection data. This may allow information obtained during the first portion's decomposition to be utilized in the second portion's decomposition, thus making the second portion's decomposition more efficient.

According to another aspect of the invention, there is provided a computer program product comprising computer program code means which, when executed on a computing device having a processing system, cause the processing system to perform all of the steps of the computer-implemented method of any herein disclosed method.

According to another aspect of the invention, there is provided a CT system. The CT system comprising: a rotatable gantry; and an X-ray tube configured to continuously vary its kVp level during rotation of the gantry but still fast enough such that each voxel of a scanned volume is scanned under at least two different kVp levels, wherein a monotonic rate of change between two extrema kVp levels is at most 5kVp/ms.

In some embodiments, the CT system may further comprise an adjustable filter in a collimator box of the CT system configured to controllably filter beams of different kVp levels.

Thus, there may be proposed concepts for operating a CT system, and this may be done based on slowly varying a kVp level of an X-ray tube such that every voxel of a scanned volume will be seen under at least two different kVp levels, thus enabling spectral CT imaging without requiring any specialized hardware.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for operating a CT system according to a proposed embodiment;
Fig. 2 is an example illustration of a variation of a slow kVp level change during a gantry rotation according to a proposed embodiment;
Fig. 3 is a flow diagram of a method for operating a CT system according to a proposed embodiment;
Fig. 4 is a simplified block diagram of a CT system according to a proposed embodiment; and
Fig. 5 illustrates an example of a computer within which one or more parts of an embodiment may be employed.

### DETAILED DESCRIPTION OF THE EMBODIMENTS

The invention will be described with reference to the Figures.

Embodiments of the invention aim to provide a method for operating a CT system. This can be achieved by slowly varying a kVp level of an X-ray tube of the CT system (as opposed to having to use an expensive fast-switching kVp generator, for example) so that each voxel of the scanned volume is scanned/imaged under at least two different kVp levels.

In other words, by slowly varying the kVp level of an X-ray tube during rotation of the gantry of the CT system, every voxel of a scanned volume can be seen under at least two different kVp levels, thus enabling spectral CT imaging without any specialized hardware, thus facilitating spectral imaging even in a standard, one source, one detector CT system.

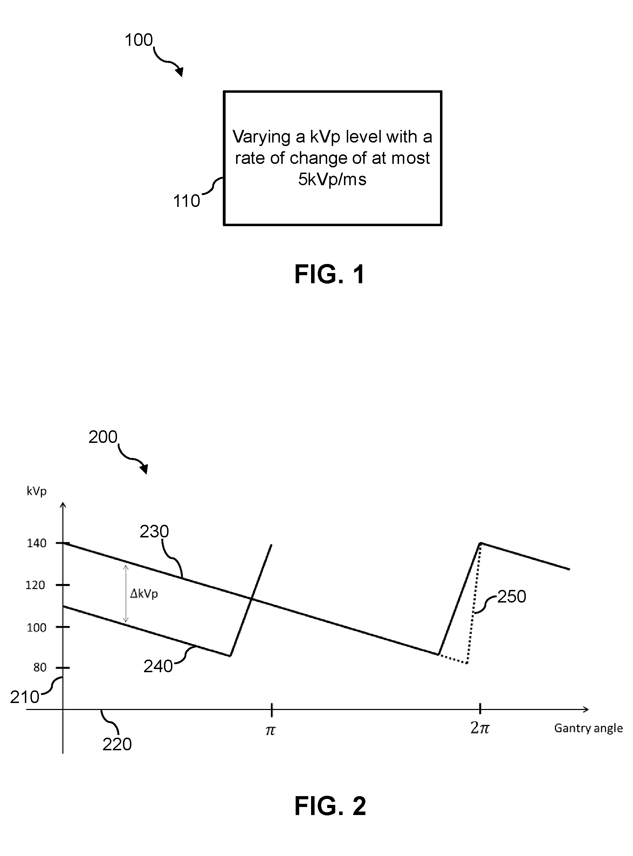

Referring now to Fig. 1, there is depicted a simplified flow diagram of a method 100 for operating a CT system according to a proposed embodiment.

The method 100 comprises step 110 of, during rotation of a gantry of the CT system, slowly varying a kVp level of an X-ray tube of the CT system but still fast enough such that each voxel of a scanned volume is scanned under at least two different kVp levels during a single rotation.

A monotonic rate of change can be understood as a uni-directional rate of change of the kVp level, i.e., a rate of change in a single direction, i.e., the rate of change while maintaining a consistent direction of change, when the kVp level oscillates between two kVp levels (a maximum and a minimum). The rate of change can be expressed as kVp per rotation or as kVp per time period. In Fig. 1, the kVp level falls from 140kVp to 85kVp in an almost full gantry rotation of 11/6 π (corresponding to a monotonic rate of change of 60 kVp / rotation) and rises back to 140 kVp in another part of the gantry rotation of 1/6 π (a monotonic rate of change of 660 kVp / rotation). At a gantry rotation of 5 Hz, the fall rate of change in this example would be 300 kVp/s and the rise rate of change would be 3300 kVp/s. In contrast, fast kVp switching systems require much higher fall rates and rise rates, of around 3 orders of magnitude more, as the kVp level has to switch between low and high kVp at a much higher (sampling) frequency.

In this embodiment, the CT system is a single-source and single-detector CT system. This enables the method to be used even on the cheapest and most widely-available CT systems, where the present invention will have the most benefit. In other embodiments, however, as would be understood by the skilled person, the CT system can comprise any type of CT system, even those capable of performing spectral imaging in one of the traditional ways.

Furthermore, in this embodiment, the variation of the kVp level comprises a period of decreasing and a period of increasing, and the period of decreasing is longer than the period of increasing. Decreasing the kVp level of an X-ray tube is dependent on the emission current, and thus its rate of change is limited compared to increasing the kVp level, which is essentially independent of the emission current. By letting the kVp level slowly decrease but then more rapidly increasing the kVp level (back up to the maximum kVp, for example) the number of views obtained with a similar kVp can be reduced, thus increasing the efficiency and utility of the scan.

Specifically, in this embodiment, the kVp level of the X-ray tube is varied in a sawtooth pattern. This is a simple pattern to implement for the varying of the kVp level and provides a more uniform variation of different kVp levels. Of course, as would be understood by the skilled person, in other embodiments, the kVp level can be varied in any other suitable pattern, e.g., a triangle wave pattern, a ramp wave pattern, a pulse wave pattern, a sine wave pattern, a curved waveform, etc.

In this embodiment, the kVp level varies by at least 10k Vp. This ensures a minimum variation of kVp levels, thus facilitating more useful spectral imaging and hence more useful material decomposition. In other embodiments, however, the kVp level can vary by at least any other suitable value, for example, 20kVp, 30kVp, 40kVp, 50kVp, 60kVp, 70kVp, 80kVp, 90kVp, or 100kVp. Having a minimum variation allows for sufficiently good spectral performance and signal-to-noise ratio.

In some embodiments, the kVp level of the X-ray tube can be varied in a pattern with a frequency that is different to the frequency of the gantry rotation. This can enable better sampling with different kVp levels, however, is not essential to the working of the invention. For example, in this embodiment, the kVp-modulation cycle has the same frequency as the gantry rotation. Furthermore, in this embodiment, the phase of the kVp-modulation cycle is the same as the gantry rotation, i.e., they start at the same time and end at the same time, however, in other embodiments, the kVp-modulation and the gantry rotation can be out of phase with each other, i.e., they can have a phase shift between them.

In other words, in some embodiments, the variation of the kVp level can be out of sync with the gantry rotation, i.e., there can be a difference in period between the gantry rotation (i.e., the time to complete a full gantry rotation) and the pattern with which the kVp level of the X-ray tube varies (e.g., the period from maximum kVp to maximum kVp again). For example, in some embodiments, it can be advantageous to have the kVp-modulation frequency lower than the frequency of the gantry rotation if a helical acquisition with a small pitch is performed. Even if the periods/frequencies of the kVp-modulation and the gantry rotation are different, however, they should be within a certain distance of each other. For example, the period of the pattern with which the kVp level varies should be within 0.5 to 5 times the period of a single gantry rotation (or, in other words, have a frequency within 0.2 to 2 times the gantry rotation frequency).

It should be noted that the single rotation of the gantry of the CT system can be during a helical scan or an axial scan. These are both types of CT scans where the present invention would be of benefit.

Referring now to Fig. 2, there is depicted an example illustration of a variation of a kVp level during a gantry rotation according to a proposed embodiment.

The illustration comprises a graph 200 with gantry angle on the x-axis 200, and kVp level on the y-axis 210. Line 230 shows the variation of the kVp level of an x-ray tube of a CT system, slowly decreasing from 140kVp to just over 80kVp over nearly a full gantry rotation before (relatively) quickly increasing back up to 140kVp for the end of the gantry rotation, to then, for example, begin again in the next rotation. This is a sawtooth pattern.

In other words, line 230 shows the kVp level during a gantry rotation with angle values in units of pi. Rays from angles with a difference of pi go through the same path but with opposite directions (i.e., are pi-partners). Line 240 is thus the part of line 230 starting from pi and going to 2pi, but phase-shifted back by pi. This is purely for illustrating the kVp differences for rays from angles with a difference of pi (i.e., between pi-partners). This kVp difference is important for material decomposition, and as can be seen, in this example, a constant difference between the pi-partners of 30 kVp is realized.

Line 250 is purely for illustrating an alternative sawtooth pattern, wherein the period of decreasing is longer and then the period of increasing is shorter, i.e., the kVp increases back up to the maximum (from a lower minimum) quicker.

Varying the kVp level according to a sawtooth pattern, such as that shown in the graph 200 is favorable for a number of reasons. First, a standard X-ray generator providing the acceleration voltage between the cathode (filament) and the tungsten anode is typically equipped with a smoothing capacity to reduce the kVp-ripple in a constant kVp mode. This limits the kVp-modulation capabilities of the generator. An increase of kVp is always possible within a short time period, e.g., a few tens of microseconds, but a reduction of kVp is only possible via the discharge of the emission current (producing the X-rays) as the generator is designed only to increase the kVp. Thus, the reduction rate of the kVp linearly depends on the emission current. The decrease of kVp can be on the order of about a few ms per 10kVp (as stated, the monotonic rate of change should be at most 5kVp/ms), whereas the increase of kVp is almost independent of the emission current.

Secondly, the modulation of the emission current is also bandwidth limited to roughly 1.5% per ms with a maximum of about 5mA-10mA per ms. A reduction/increase of kVp corresponds to a reduction/increase of the emission current. A slow kVp-modulation thus has the advantage that the occurring X-ray dose drop when reducing the kVp level can be compensated/diminished by increasing the emission current.

The modulation of the kVp and the emission current can be accomplished without hardware changes of the CT systems besides, perhaps, control printed circuit boards (PCBs), which might be replaced to enable setting new kVp levels during the scan. A method to calculate the patient-specific dose modulation with slow kVp modulation would also be helpful but is a simple optimization task, easily appreciated and accomplishable by the skilled person without any undue burden.

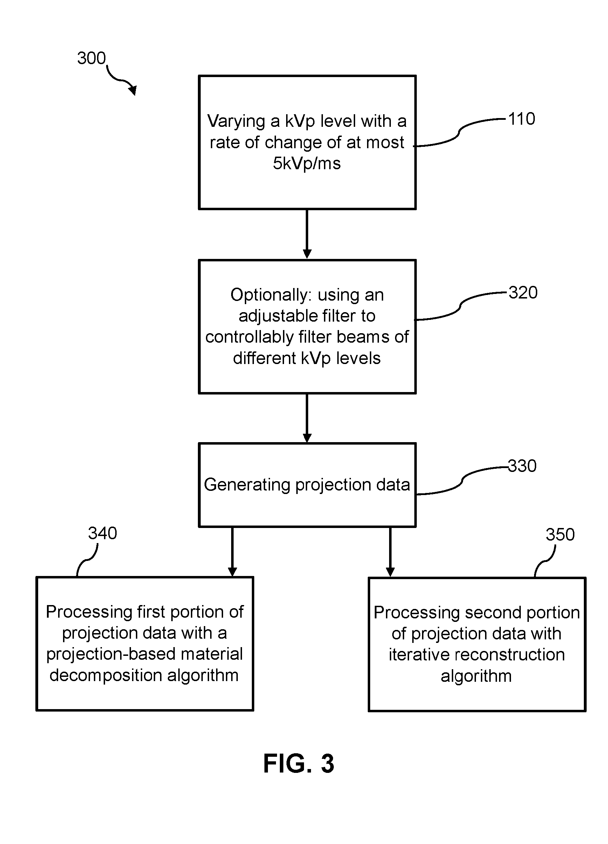

Referring now to Fig. 3, there is depicted a flow diagram of a method 300 for operating a CT system according to a proposed embodiment. Step 110 is substantially the same as has been described in relation to method 100 of Fig. 1.

Step 320 comprises using an adjustable filter in a collimator box of the CT system to controllably filter beams of different kVp levels. This minor hardware modification can further improve the spectral performance of the proposed method. For example, the adjustable filter can comprise a movable filter which, for example, can be a metal wedge movable in and out of the X-ray beam such that the high kVp beams are filtered by more material than the low kVp beams. Of course, as would be understood by the skilled person, this step 320 would take place in synchronization with the variation of the kVp level in step 110, but is not essential for the working of the invention.

In other words, the noise in material decomposition results depends on the noise in the input data and the differences in energy used (e.g., the difference in kVp levels for pi-partners). The X-ray spectra of different kVp levels overlap at low energies, as the skilled person would appreciate. Thus, by using an adjustable filter to filter the high kVp levels, the number of low energy photons can be reduced in the high kVp spectra. By doing this, the spectral overlap between the spectra of different kVp levels is reduced, and thus the energy difference between the spectra is increased, thereby reducing noise in the material decomposition results and also reducing patient dose.

In step 330, projection data is generated based on the volume scanned in step 110.

A first portion of the projection data is then processed in step 340. The first portion of the projection data corresponds to (at least one, but preferably all the) pairs of detector measurements along the same paths but in opposite directions. The processing is performed with a projection-based material decomposition algorithm to decompose the first portion of the projection data into at least two different materials. In other words, for a pair of detector measurements transversing the same path in opposite directions, also known as pi-lines or pi-partners, projection-based decomposition can be applied directly, facilitating more efficient material decomposition. Thus, preferably, all pi-lines or pi-partners should be processed with the projection-based material decomposition algorithm to decompose the projection data corresponding to the pi-lines into at least two different materials.

In some embodiments, the first portion of the projection data can also include approximated pi-lines for pairs of detector measurements which are within a predetermined distance of a central detector slice of the projection data.

In step 350, a second portion of the projection data is processed with an iterative spectral reconstruction algorithm to decompose the second portion of the projection data into at least two different materials. This enables all detector measurements to be used for material decomposition, regardless of whether they are pi-lines or pi-partners. In this embodiment, the second portion of the projection data is the projection data minus the first portion of the projection data, i.e., the projection data without the pi-lines. In other words, in this embodiment, the projection data consists of the first portion and the second portion, with no overlap between the portions. In other embodiments, however, there can be some overlap between the portions. Alternatively, for example, the second portion of the projection data can comprise all of the projection data (and step 340 can be ignored, for example).

An iterative spectral reconstruction algorithm is a computational method that iteratively refines an estimate of a signal's spectrum using observed data and a mathematical model, aiming to recover the underlying spectral content (or more specifically, the underlying attenuation of the object). It repeatedly adjusts the estimated attenuation until it converges to a solution that best fits the observed data, enabling the reconstruction of spectral features from incomplete or noisy measurements. It can thus be understood as performing both material decomposition and image reconstruction in `one' step.

In this embodiment, processing the second portion of the projection data is at least partly based on the decomposition of the first portion of the projection data. This allows information obtained during the first portion's decomposition to be utilized in the second portion's decomposition (e.g., as a prior), thus making the second portion's decomposition more efficient, but as the skilled person would understand, is not essential to the working of step 350 or the method 300 as a whole.

Of course, if the varying of the kVp level in step 110 is such that each voxel of the scanned volume is scanned under n (n being more than or equal to 2) kVp levels, then the material decomposition in steps 340 and 350 can be into n different materials.

In a simple example of an axial scan utilizing the concepts of the present invention, a kVp modulation cycle is implemented as shown in Fig. 2. Central detector slice pi-lines exist for each detector in the CT system and an exact projection-based material decomposition can be conducted for said pi-lines. For rays close to the central detector slice, the pi-lines can be approximated. However, for detector slices far away from the central detector slice, iterative reconstruction is required (as in step 350).

In another example, almost every voxel is irradiated by at least n (e.g., n=3) kVp levels and almost every pi-line for the central detector slice is illuminated by n different kVp levels allowing for a material decomposition into n materials or for a statistically weighted material decomposition into m<n different materials. In this example, projection data over more than one gantry rotation (e.g., 1.5 or 2) may be acquired. The kVp schedule can thus be optimized to achieve the best possible conditioning of the material decomposition problem at hand.

For example, in order to materially decompose into a larger number of different materials, more rotations per step can be performed and/or the rate with which the kVp varies can be decreased such that the full period over which the kVp varies might be more than one full gantry rotation. For example, instead of the period of decreasing lasting over nearly a single gantry rotation (as seen in Fig. 2), the period of decreasing can last over nearly two whole gantry rotations which could thus allow n=4 (i.e., material decomposition into 4 different materials). In other words, for n=4, the period of the pattern with which the kVp level is varied can be (either exactly or close to) two full gantry rotations, and for n=3, the period of the pattern with which the kVp level is varied can be (either exactly or close to) 1.5 full gantry rotations.

To be clear, for every half a gantry rotation that the kVp level is decreasing over, there is provided another kVp level with which the same voxels are scanned under (and which have a constant energy difference with their pi-partners if the rate of decreasing is kept constant). Therefore, if the kVp level is uniformly decreased over two full rotations of the gantry, then every voxel will be scanned under at least four different energies (with uniform energy differences), thus allowing material decomposition into four materials.

In these embodiments, of course, the variation of the kVp level during a single gantry rotation might not include both a period of increasing and decreasing but perhaps only a period of decreasing, with the period of increasing occurring in a second or third gantry rotation, for example.

In a simple example of a helical scan utilizing the concepts of the present invention, a small pitch is selected to scan each voxel by at least two rays (e.g., after wedge re-binning). A parallel re-binning of the multi-kVp data can be accomplished by interpolating data of different kVps and re-setting the kVp level for this ray (e.g., interpolation of rays with 100kVp and 103kVp and setting its nominal kVp to 101.5kVp). In parallel geometry, a projection-based material decomposition for rays from opposite directions is possible. Each parallel view consists of rays with different kVp-levels. Using a small pitch and an appropriate kVp modulation scheme, each pi-line/voxel is illuminated by at least n (e.g., n=4) kVp levels allowing for a material decomposition into n materials or for a statistically weighted material decomposition into m<n different materials. In addition, an iterative reconstruction in native fan beam geometry can be used, avoiding interpolation.

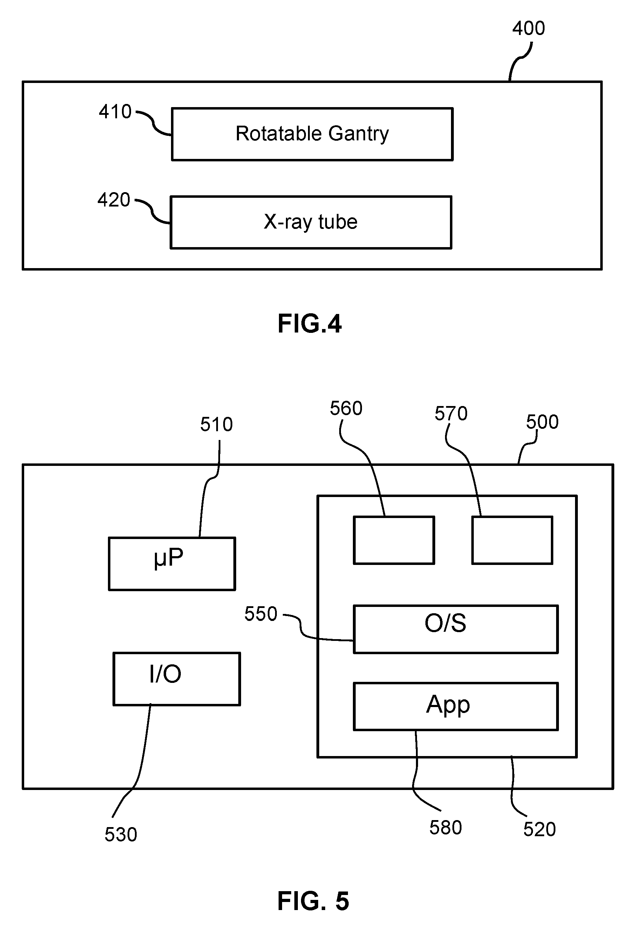

Referring now to Fig. 4, there is depicted a simplified block diagram of a CT system 400 according to a proposed embodiment.

The CT system 400 comprises a rotatable gantry 410. A rotatable gantry in a CT system allows for the X-ray tube 420 and detector array (not shown) to rotate around the patient or object being scanned, capturing multiple views from different angles. This rotation enables the acquisition of data from various perspectives.

As mentioned, the CT system 400 also comprises an X-ray tube 420 (e.g., mounted on the rotatable gantry 410) configured to continuously vary its kVp level during a single rotation of the gantry such that each voxel of a scanned volume is scanned under at least two different kVp levels, wherein a monotonic rate of change of the kVp level is at most 5kVp/ms

Of course, as the skilled person would understand, the CT system 400 would also comprise all of the standard components of a CT system not explicitly depicted, such as, for example, one (or more) detector array(s) positioned opposite the X-ray source (on the rotatable gantry) to capture the transmitted radiation; a patient table used to position the object/subject within the scanner, ensuring accurate alignment during the scanning process; a computer system for processing the raw data collected by the detectors to reconstruct cross-sectional images of the scanned object using algorithms such as filtered back projection or iterative reconstruction; and a display monitor to visualize, for example, the reconstructed images.

In this embodiment, the CT system is a single-source and single-detector CT system, however, in other embodiments, this need not be the case.

In some embodiments, the CT system 400 can further comprise an adjustable filter in a collimator box of the CT system 400 to controllably filter beams of different kVp levels. As mentioned above in relation to method 300 of Fig. 3, this minor hardware modification can further improve the spectral performance of the proposed method. For example, the adjustable filter can comprise a movable filter, which for example, can be a metal wedge movable in and out of the X-ray beam such that the high kVp beams are filtered by more material than the low kVp beams.

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to operating a CT system. According to proposed concepts, a number of possible solutions may be implemented separately or jointly. That is, although these possible solutions may be described below separately, two or more of these possible solutions may be implemented in one combination or another.

## Claims

1. A computer-implemented method (100) operating a CT system, the method comprising:
during a single rotation of a gantry of the CT system, slowly varying a kVp level of an X-ray tube (110) of the CT system but still fast enough such that each voxel of a scanned volume is scanned under at least n kVp levels, where n is 2 or more.

2. The computer-implemented method of claim 1, wherein the rate of change of the kVp level during the slow variation is at most 5kVp/ms.

3. The computer-implemented method of claim 1 or 2, wherein the CT system is a single-source and single-detector CT system.

4. The computer-implemented method of claims 1 to 3, wherein the variation of the kVp level comprises a period of decreasing and a period of increasing, and wherein the period of decreasing is longer than the period of increasing.

5. The computer-implemented method of any of claims 1 to 4, wherein the kVp level of the X-ray tube is varied in a sawtooth pattern.

6. The computer-implemented method of any of claims 1 to 5, wherein the kVp level varies by at least 10kVp.

7. The computer-implemented method of any of claims 1 to 6, wherein the kVp level of the X-ray tube is varied in a pattern with a frequency that is different to the frequency of the gantry rotation.

8. The computer-implemented method of any of claims 1 to 7, wherein the method further comprises using an adjustable filter (320) in a collimator box of the CT system to controllably filter beams of different kVp levels.

9. The computer-implemented method of any of claims 1 to 8, wherein the single rotation of the gantry of the CT system is during a helical scan or an axial scan.

10. The computer-implemented method of claim 9, wherein the method further comprises: generating projection data (330) based on the scanned volume, and processing a first portion of the projection data (340), corresponding to pairs of detector measurements along the same path but in opposite directions, with a projection-based material decomposition algorithm to decompose the first portion of the projection data into at least two different materials.

11. The computer-implemented method of claim 9 or 10, wherein the method further comprises:
processing a second portion of the projection data (350) with an iterative spectral reconstruction algorithm to decompose the second portion of the projection data into at least two different materials.

12. The computer-implemented method of claim 11, dependent on claim 10, wherein processing the second portion of the projection data is at least partly based on the decomposition of the first portion of the projection data.

13. A computer program product comprising computer program code means which, when executed on a computing device having a processing system, cause the processing system to perform all of the steps of the computer-implemented method according to any of claims 1 to 12.

14. A CT system (400) comprising:
a rotatable gantry (410); and
an X-ray tube (420) configured to slowly vary its kVp level during rotation of the gantry but still fast enough such that each voxel of a scanned volume is scanned under at least n different kVp levels, where n is 2 or more.

15. The CT system of claim 14, further comprising an adjustable filter in a collimator box of the CT system configured to controllably filter beams of different kVp levels.
